# EUROPÄISCHE PATENTSCHRIFT

(11) **EP 0 697 106 B1**
(45) Veröffentlichungstag und Bekanntmachung des Hinweises auf die Patenterteilung: **02.04.1997**
(21) Anmeldenummer: 94915552.7
(22) Anmeldetag: 27.04.1994
(51) Int. Cl.: G01N 21/66, G01N 33/52, G01N 33/58

(54) **ELEKTROCHEMILUMINESZENZVERFAHREN**
ELECTROCHEMILUMINESCENT PROCESS
PROCEDE ELECTROCHIMILUMINESCENT

(30) Priorität: 03.05.1993 DE 4314547; 25.09.1993 DE 4332697; 20.01.1994 DE 4401577
(43) Veröffentlichungstag der Anmeldung: 21.02.1996
(73) Patentinhaber: BOEHRINGER MANNHEIM GMBH, 68298 Mannheim (DE)
(72) Erfinder: KLEMT, Volker, D-82362 Weilheim (DE); MÜLLER, Günter, D-82380 Peissenberg (DE); NEUMANN, Ulrich, D-82362 Weilheim (DE); GIESEN, Ursula, D-82362 Weilheim (DE); HOYLE, Nicholas, D-82327 Tutzing (DE)
(86) Internationale Anmeldenummer: EP9401328
(87) Internationale Veröffentlichungsnummer: WO9425853

(56) Entgegenhaltungen:
- EP-A- 0 096 749
- WO-A-90/05296
- WO-A-90/05302
- US-A- 4 927 769
- JOURNAL OF PHOTOCHEMISTRY AND PHOTOBIOLOGY, Bd.54, Nr.3, 1990, LAUSANNE Seiten 311 - 319 H. KARATANI ET AL. 'ELECTROGENERATED CHEMILUMINESCENCE OF CYCLIC HYDRAZIDES IN AN ALKALINE BRIJ35 MICELLAR SYSTEM.'

## Beschreibung

Gegenstand der Erfindung sind Verfahren zur Messung elektrochemilumineszenter Phänomene, Verfahren zum Nachweis eines Analyten mittels solcher Verfahren, Reagenzlösungen, die in diesen Verfahren eingesetzt werden können und ein für die Durchführung des Verfahrens besonders geeigneter Apparat.

Verfahren zur Messung elektrochemilumineszenter Phänomene sind seit einigen Jahren bekannt. Bei solchen Verfahren wird die Fähigkeit von speziellen Metallkomplexen ausgenutzt, durch Oxidation in einen Zustand zu gelangen, von dem aus sie unter Abgabe von elektromagnetischer Strahlung in einen Grundzustand zurückfallen. Solche Verfahren und dafür geeignete Metallkomplexe sind beispielsweise in der WO 86/02734 beschrieben.

Diese Technologie wurde immer weiter verfeinert. In der WO 90/05296 wird der Testzusammensetzung ein Amin zugesetzt, bevorzugt Tripropylamin, das in oxidierter Form ein starkes Reduktionsmittel darstellt. Die elektrochemische Reaktion findet in einem Elektrolyten statt, in dem der Elektrochemielumineszenz (ECL)-Rest, d. h. der zur Abgabe von elektromagnetischer Strahlung befähigte Metallkomplex, und das Amin oxidiert werden können. Als geeigneter Elektrolyt in wässriger Lösung wird Phosphatpuffer bei einem pH von 6 - 9, bevorzugt 7 - 7,5, beschrieben.

In der WO 90/05302 wird zu dieser Testzusammensetzung zur Erhöhung der elektromagnetischen Strahlung das Detergenz Triton X-100 oder Triton N-401 zugesetzt. In der WO 90/05411 wird eine Verbesserung des Apparates zur Messung von ECL beschrieben.

Ferner gelang es, die Technologie zum Nachweis von Analyten einzusetzen, indem Elektrochemilumineszenzmarkierungen an Analyten, Analytanaloge oder analytspezifische Substanzen gekoppelt wurden. Die Elektrochemilumineszenz wurde zur Bestimmung der Menge des anwesenden Analyten verwendet. Insbesondere wurden Immunoassays beschrieben, bei denen die üblichen Markierungen durch elektrochemilumineszente Markierungen ersetzt sind.

Weitere Verbesserungen und Anwendungen der Technologie sind in WO 87/06706, WO 89/04392, WO 89/10552, WO 89/10551, WO 90/05301 und WO 90/11511 beschrieben. Die Offenbarungen in den genannten Publikationen werden im folgenden vorausgesetzt.

Aufgabe der vorliegenden Erfindung war es, die vorbekannten Verfahren zu verbessern, insbesondere im Hinblick auf die Empfindlichkeit der Analytnachweise, die mit der Elektrochemilumineszenztechnologie möglich sind.

Gegenstand der Erfindung ist ein Verfahren zur Messung elektrochemischer Phänomene in einer Lösung oder an einer an eine Lösung angrenzende feste Phase durch Inkontaktbringen einer zur Elektrochemilumineszenz fähigen Komponente, mit einem oxidierbaren Amin und Anlegen einer elektrischen Spannung zur Induktion der Elektrochemilumineszenz und Detektion der elektromagnetischen Strahlung, wobei die Lösung ein Detergenz ausgewählt aus der Gruppe der Fettalkoholethoxylate, Plantaren und Octylglucosid oder ein Gemisch hiervon enthält.

Ferner ist Gegenstand der Erfindung ein Verfahren zum Nachweis eines Analyten mit Hilfe dieser Verfahren sowie geeignete Reagenzien zur Durchführung des genannten Verfahrens.

Bei dem Erfindungsgegenstand handelt es sich um eine Lehre, die auf dem oben genannten Stand der Technik aufbaut. Die allgemeinen Grundlagen elektrochemilumineszenter Verfahren sind in diesem Stand der Technik ausführlich beschrieben. Geräte zur Durchführung von Messungen der Elektrochemilumineszenz enthalten eine Meßeinheit mit einem Behälter für eine Reagenzlösung, mindestens zwei Elektroden (eine Arbeits- und eine Gegenelektrode), die während der Messung mit der Reagenzlösung in Kontakt stehen und einen Detektor zur Messung des durch die Elektrochemilumineszenz erzeugten Lichtes. Generell wird bei diesen Verfahren zunächst an die Lösung eine Ausgangsspannung (Präpolarisation) angelegt. Anschließend wird diese Spannung über das Redoxpotential einer in der Lösung enthaltenen Substanz, z. B. einem Amin, erhöht. Über die dadurch oxidierte Substanz wird ein zur Chemilumineszenz fähiges Material, z. B. bestimmte Ruthenium-Komplexe zur Aussendung von Licht angeregt. Das innerhalb einer bestimmten Zeit vom Detektor aufgefangene Licht ist ein Maß für die Anwesenheit der Menge des elektrochemilumineszenten Materials. Sofern es sich bei dem elektrochemilumineszenten Material um eine Markierung für einen Analyten, einen Analytanalogen oder eine analytspezifische Substanz, z. B. in einem Immunoassay, handelt, ist das empfangene Licht ein Maß für die Anwesenheit des Analyten.

Es hat sich gezeigt, daß das aus der WO 90/05302 bekannte und bisher üblicherweise zugesetzte Detergenz Triton X-100, das in der Praxis meist in Kombination mit dem Detergenz Tween 20 eingesetzt wurde, nicht optimal ist. Einerseits ist Triton X-100 schlecht abbaubar und damit nicht umweltverträglich. Zum anderen wurde überraschenderweise gezeigt, daß ganz bestimmte andere Detergenzien im Vergleich zu Triton X-100 zur Verbesserung des ECL-Verfahrens führen. Mit diesen speziellen Detergenzien wird eine erhöhte Signalausbeute, ein besseres Signal/Rausch-Verhältnis und damit eine erhöhte Sensitivität des Nachweisverfahrens und eine Erniedrigung der unteren Nachweisgrenze sowie eine bessere Präzision erreicht.

Als geeignet haben sich die Detergenzien ausgewählt aus der Gruppe der Fettalkoholethoxylate, worunter zum Beispiel Polidocanol (Dodecylpoly-(ethylenglycolether)ₙ), C14-E09 (Poly(ethylenglycol-ether)ₙ), Genapol (Isotridecylpoly(ethylenglycolether)ₙ), C8-E09 (Octylalkoholpoly(ethylenglycolether)ₙ) zu verstehen sind, Plantaren® (Alkylpolyglucosid) und Octylglucosid (Octyl-beta-D-glucopyranosid) oder ein Gemisch hiervon, erwiesen. Die Detergenzien werden in Konzentrationen von 0,001 bis 1,0 % eingesetzt. Die am besten geeignetste Konzentration kann für jedes Detergenz leicht ermittelt werden. Konzentrationen von 0,1 bis 0,5 % haben sich am geeignetsten erwiesen.

Zur Konservierung wurde bisher der Testzusammensetzung Natriumazid üblicherweise in einer Konzentration von 5 - 10 mM zugesetzt. Es hat sich gezeigt, daß dieses umweltschädliche Agens durch Bioban oder Oxaban ersetzt werden kann, die weitaus umweltverträglicher als Azid sind.

Überraschenderweise hat sich gezeigt, daß diese Konservierungsmittel einen weiteren positiven Effekt auf das ECL-Verfahren ausüben. Im Vergleich zu Azid wird eine Erhöhung des Messignals beobachtet. Oxaban und Bioban werden dabei in Konzentrationen von 0,01 bis 1 %, bevorzugt 0,1 bis 0,5 % eingesetzt.

Das erfindungsgemäße Verfahren zur Messung elektrochemischer Phänomene in einer Lösung oder an einer an eine Lösung angrenzenden festen Phase kann bei Temperaturen durchgeführt werden, die über dem Gefrierpunkt der Lösung liegen, aber niedriger als 40°C sind.

Eine weitere Verbesserung der Empfindlichkeit kann erreicht werden, indem an die Meßeinheit eine Spannung angelegt wird, deren Verlauf über die Zeit rechteckig ist. Dies bedeutet, daß ausgehend von der Ausgangsspannung ein unmittelbarer Anstieg der Spannung (innerhalb maximal 0,4 Sekunden) auf die Endspannung vorgenommen wird. Für die Anregungszeit wird diese Spannung im wesentlichen konstant gehalten. Nach dieser Zeit wird die Spannung wieder unmittelbar auf eine Spannung unter dem Redoxpotential des Systems zurückgeführt. Durch diese Maßnahme ergibt sich außerdem eine Verbreiterung des dynamischen Meßbereiches, das heißt, des Bereiches, innerhalb dem Analytkonzentrationen eines festgelegten Immunoassays gemessen werden können. Für den Fall niedriger Temperaturen ist ein Salzzusatz bevorzugt.

Es hat sich als vorteilhaft erwiesen, die an die Arbeitselektrode angelegte Rechteck-Endspannung (verglichen mit Ag/AgCl) auf einen Maximalwert zwischen dem Redox-Potential der oxidierbaren Substanz und 2,2 Volt zu begrenzen. Besonders bevorzugt ist eine Spannung zwischen 1,2 - 2,2 Volt. Besonders bevorzugt sind 1,4 Volt. Diese Werte gelten für die Verwendung eines Platin- bzw. GoldElektrodenpaars.

Falls die bisher übliche Rampenspannung, beispielsweise eine Dreieckspannung, angelegt wird, wird die Endspannung (verglichen mit Ag/AgCl) auf einen Maximalwert von 3,0 Volt begrenzt.

Überraschenderweise konnte auch eine Erhöhung des Signals erreicht werden, indem die Ausgangsspannung vor Anregung der Elektrochemilumineszenz an der Arbeitselektrode zwischen + 400 und - 400 mV, verglichen mit einer Ag/AgCl-Elektrode, betrug. Besonders bevorzugt ist ein Wert zwischen + 0 mV und + 200 mV. Auch hier gelten die Werte für die Verwendung eines Platin- bzw. Goldelektrodenpaars. Die Potentiale für andere Elektrodenmaterialien können leicht errechnet werden.

Ebenfalls eine Steigerung des Signals läßt sich erreichen durch Einstellung eines pH-Wertes zwischen 6,5 - 9,0, bevorzugt zwischen 6,5 - 7,5, besonders bevorzugt 6,8. Dies geschieht zweckmäßigerweise durch Einsatz eines für den Bereich geeigneten pH-Puffers. Zusätzlich können noch ein oder mehrere Alkali- oder Erdalkalihalogenide in einer Konzentration von 0,05 mmol/l bis 0,5 mol/l in der Lösung enthalten sein. Bevorzugt wird Natriumchlorid eingesetzt.

Die oben genannten Maßnahmen führen alleine schon zu erheblichen Verbesserungen der bekannten Verfahren. Darüber hinaus jedoch kann die Empfindlichkeit oder/und der dynamische Meßbereich von Analytnachweisen durch eine Kombination der Maßnahmen in besonders großem Umfang gesteigert werden.

Die Steigerung der Nachweissensitivität von Analyten, beispielsweise in Immunoassays, wie nach dem Sandwich- oder kompetitiven Verfahren, erlaubt weitere Vereinfachungen des Verfahrens oder der verwendeten Apparate. Beispielsweise ist es nun möglich, als Detektor eine Photodiode zu verwenden, die Systemkalibration zu vereinfachen, wegen der geringeren Meßzeit bei erhöhtem Signal die Anzahl von durchgeführten Tests in der Zeiteinheit zu erhöhen oder die Probenvolumina zu verringern.

Ebenfalls Gegenstand der Erfindung ist eine Reagenzlösung zur Messung elektrochemischer Phänomene und insbesondere zum Nachweis von Analyten, enthaltend ein elektrochemisch oxidierbares Amin, das im oxidierten Zustand ein starkes Reduktionsmittel darstellt, welche ein Detergenz ausgewählt aus der Gruppe Fettalkoholethoxylate, Plantaren® und Octylglucosid oder ein Gemisch hiervon enthält. Zusätzlich kann noch ein Alkalichlorid in einer Konzentration von 0,1 mmol/l bis 0,5 mol/l enthalten sein und/oder der pH-Wert zwischen 6,8 - 8,0 liegen. Weiterhin können noch übliche Zusatzstoffe, wie beispielsweise Puffersubstanzen, Stabilisatoren und Konservierungsmittel, enthalten sein.

Die Reagenzlösung wird bevorzugt mit Bioban oder Oxaban konserviert.

Ein Apparat zur Durchführung von Nachweisen mittels Elektrochemilumineszenz ist beispielsweise in Beispiel 1 der WO 90/05302 ausführlich beschrieben. Ein Apparat kann darüber hinaus ein Mittel zur Kühlung der Meßeinheit oder/und eines Flüssigkeitsbehälters auf Temperaturen zwischen 0 - 25 °C enthalten, falls das Verfahren bei diesen niedrigen Temperaturen durchgeführt werden soll. Als Meßeinheit soll hier die Zelle verstanden werden, in der die Messung der Elektrolumineszenz vorgenommen wird. Unter Flüssigkeitsbehälter kann ein Vorratsgefäß oder aber eine Zuführung, z. B. Schlauch, für die während der Messung in der Meßeinheit befindlichen Reagenzlösung verstanden werden.

Ebenfalls Gegenstand der Erfindung ist ein Verfahren zum Nachweis eines Analyten über eine Elektrochemilumineszenzmarkierung, wobei eines der oben genannten Verfahren zur Messung elektrochemilumineszenter Phänomene verwendet wird.

Die folgenden Beispiele sollen die Erfindung näher erläutern:

### Beispiel 1

In einer Versuchsreihe wurde der Effekt der erfindungsgemäß eingesetzten Detergenzien ermittelt. Um den Einfluß der Detergenzien auf die Signalerzeugung möglichst unabhängig von einzelnen Testparametern, d. h. zu bestimmten Analyten bestimmen zu können, wurden streptavidinüberzogene Magnetpartikel, an denen ein biotinylierter und gleichzeitig ruthenylierter Antikörper gekoppelt war, eingesetzt (HSAP: "hot-Streptavidin-Partikel").

Zur Messung wurde ein Apparat, wie er in Beispiel 1 der WO 90/05302 beschrieben ist, welcher ferner in der Meßzelle einen Permantemagneten enthielt (Origen 1.0 von IGEN, Rockville, USA oder Magnalyser ) benutzt. Dieses Instrument enthält weiterhin einen Photomultiplier, einen Potentiostaten, eine elektrochemische Durchflusszelle, Flüssigkeitstransfermittel und eine 50-Tube Probenrotor.

Zum Nachweis wurden in einem Tube vereinigt:

| | |
|---|---|
| HSAP (lyophilisierte HSAP wurden in einem Tris/Polidocanol-Puffer (100mM;0,1%) pH 9,0 gelöst, sodaß eine Arbeitslösung von 600 µg/ml vorlag) | 50 µl |
| PBS-Puffer (50 mM KH₂PO₄; 100mM NaCl; 0,1% RSA; pH7,0) | 200 µl |
| Reagenzlösung (200 mM KH₂PO₄-Puffer; 100 mM TPA; pH 7,5; jeweilig getestetes Reagenz) | |

Dieses Gemisch wurde in ein Meßtube pipettiert und anschließend in die Meßzelle überführt. Die HSAP wurden mit dem Puffer (AB) gewaschen und in diesem Puffer die Signalausbeute vermessen.

Der verwendete Antikörper wurde mit Biotin-DDS (Biotinylamino-3,6-dioxaoctanoyl-aminocarbonyl-heptansäure-N-hydroxysuccinimidester) biotinyliert. (Tris)(2,2'-Bipyridyl) Rutheniumchloridhexahydrat wurde mit DSS (Disuccinylsuberat) an die Antikörper gebunden.

Die streptavidinüberzogene Magnetpartikel wurden von der Firma Deutsche Dynal GmbH, Deutschland bezogen (Dynabeads M-280 Streptavidin).

Der Puffer (AB), der bei der Messung verwendet wurde, hatte folgende Zusammensetzung:

| | |
|---|---|
| KH2PO4 * 2H2O | 0,2 M |
| KOH | 0,076 M |
| NaCl | 0,05 mM |
| TPA (Tripropylamin) | 0,1 M |
| Detergenz | in den in der Tabelle angegebenen Konzentrationen |
| Oxaban/Bioban | 0,1/0,3 % |
| pH | 7,5 |

Als Kontrolle wurden die bisher üblichen Detergenzien Tween 20 und Triton X-100 jeweils in einer Konzentration von 0,05 % eingesetzt. Zum Vergleich wurde in der Tabelle 1 die mit diesem Detergenz erhaltene Signalausbeute als 100 % betrachtet. Als weiterer Meßwert wurde die unspezifische Signalausbeute im Puffer (AB) bestimmt und hiermit das Verhältnis der Signalausbeute HSAP/AB ermittelt. Dieses Verhältnis zwischen der Signalausbeute mit und ohne HSAP stellt ein gutes Indiz für die Sensitivität des Testes dar. Aus den Ergebnissen der Tabelle 1 ist klar zu erkennen, daß die erfindungsgemäßen Detergenzien am geeignetsten sind.

Polidocanol und C8-E09 zeigen den besten Einfluß auf das Verhältnis HSAP/AB. Andere Detergenzien bewirken im Vergleich zu Tween/Triton X-100 eine Verschlechterung der Signalausbeute.

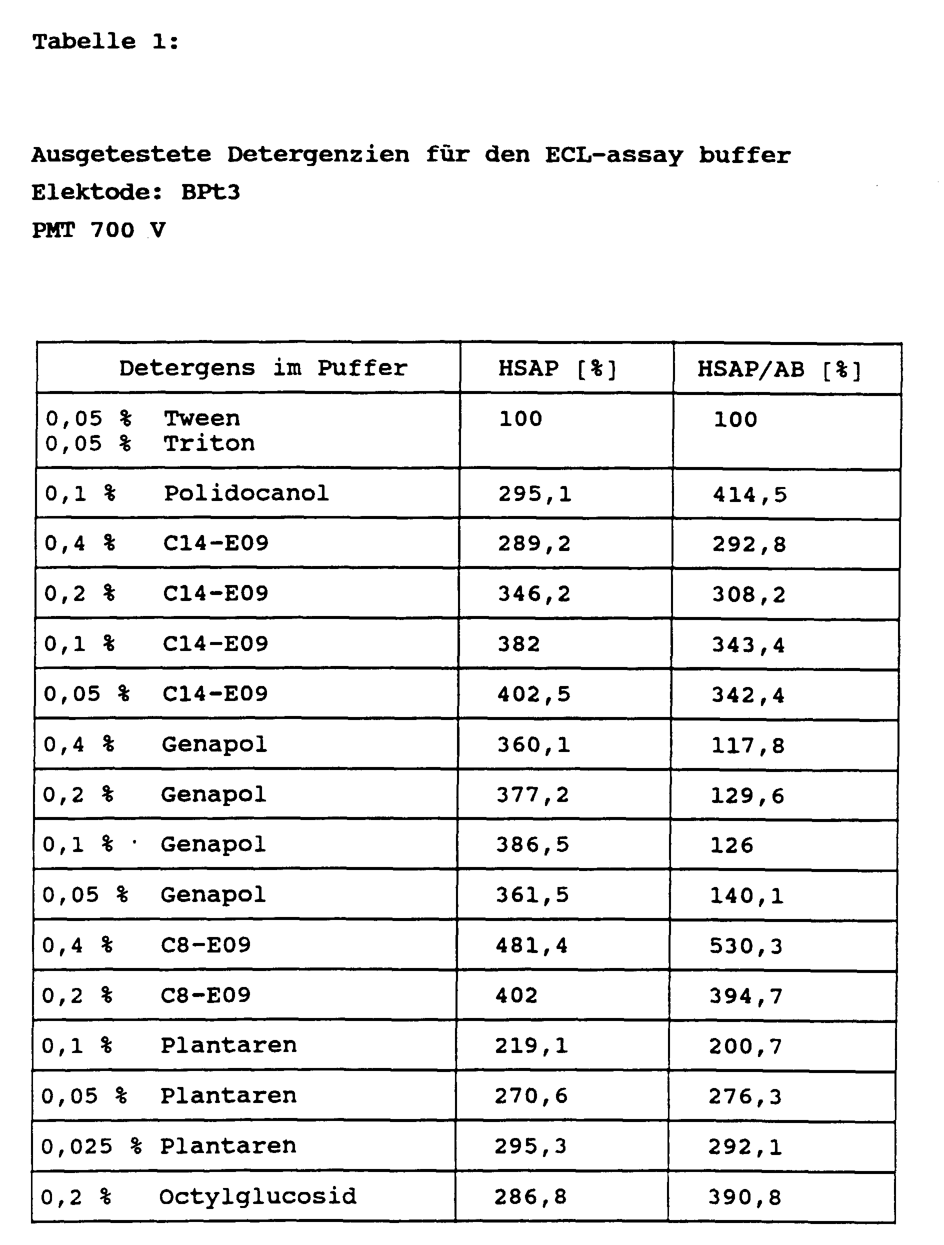

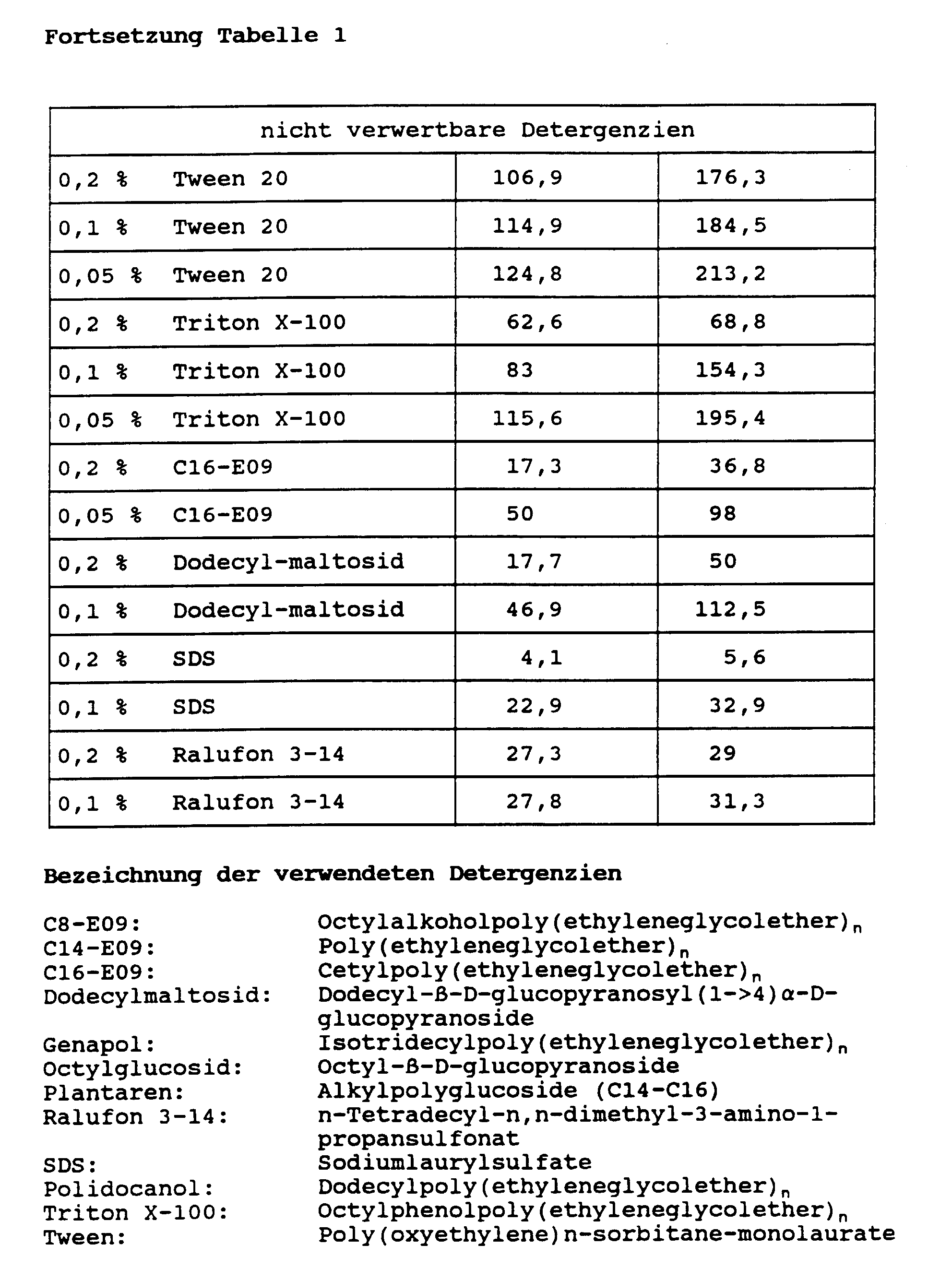

### Beispiel 2

Am Beispiel eines Parameterunabhängigen Tests (HSAP) wird die Auswirkung der Zelltemperatur auf die Signalwiederfindung und die Dynamik für den Fall einer Rampenspannung bei 28 - 35°C in der Meßzelle in Abhängigkeit von NaCl wiedergegeben.

Der Test wurde wie im Beispiel 1 gezeigt durchgeführt. Anstatt des Puffers (AB) wurde der Puffer BMG 2 mit folgender Zusammensetzung verwendet:

| | |
|---|---|
| H₃PO₄ | 0,2 M |
| Polidocanol | 0,1 % |
| Oxaban | 0,1 % |
| Tripropylamin | 0,16 M |
| KOH | 0,12 M |
| pH | 6,8 |
| NaCl | in der Tabelle angegebene Konzentration |

Die Ergebnisse sind in der Tabelle 2 dargestellt. Durch eine Erhöhung der Temperatur und der Salzkonzentration steigen die ECL-Signale, die mit Puffer (BMG2) allein und mit HSAP erhalten wurden. Die Verhältnisse HSAP/AB, HSAP/FC und FC/AB sind von der Temperatur nahezu unabhängig, dagegen von der Salzkonzentration abhängig.

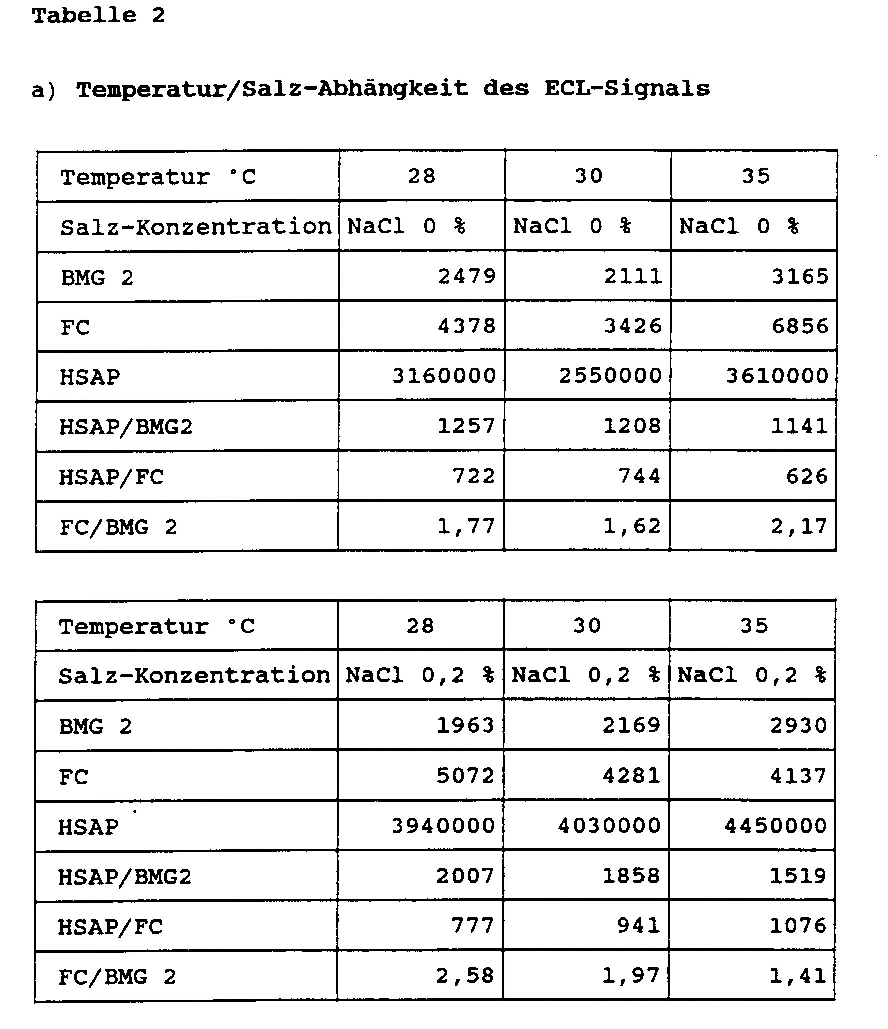

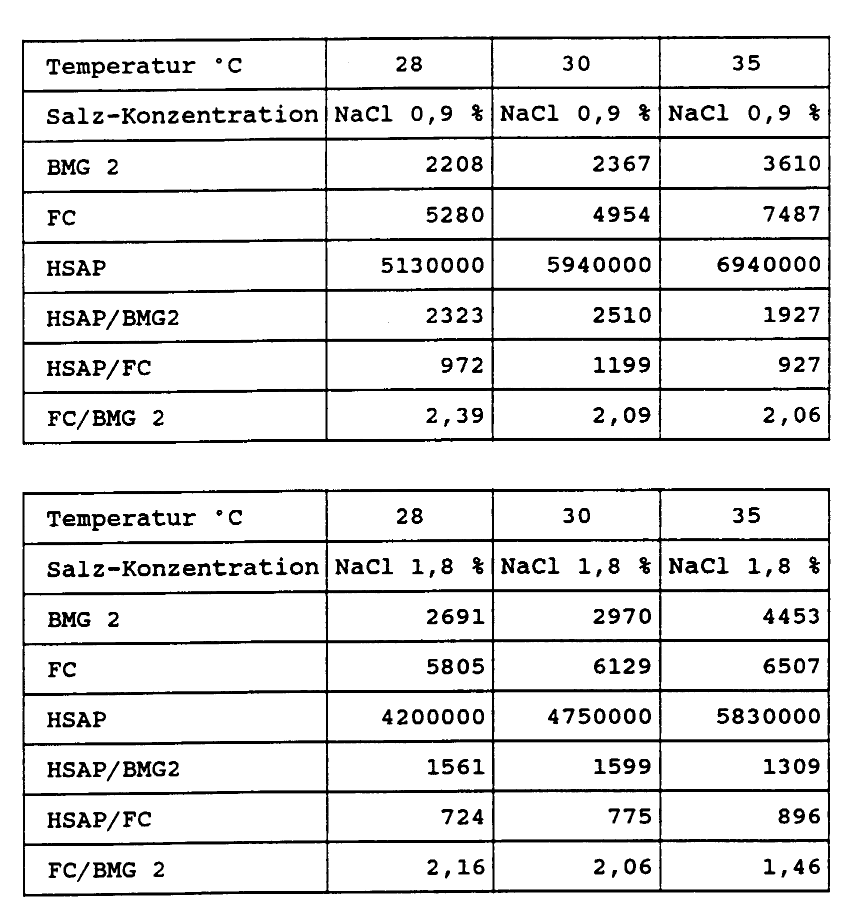

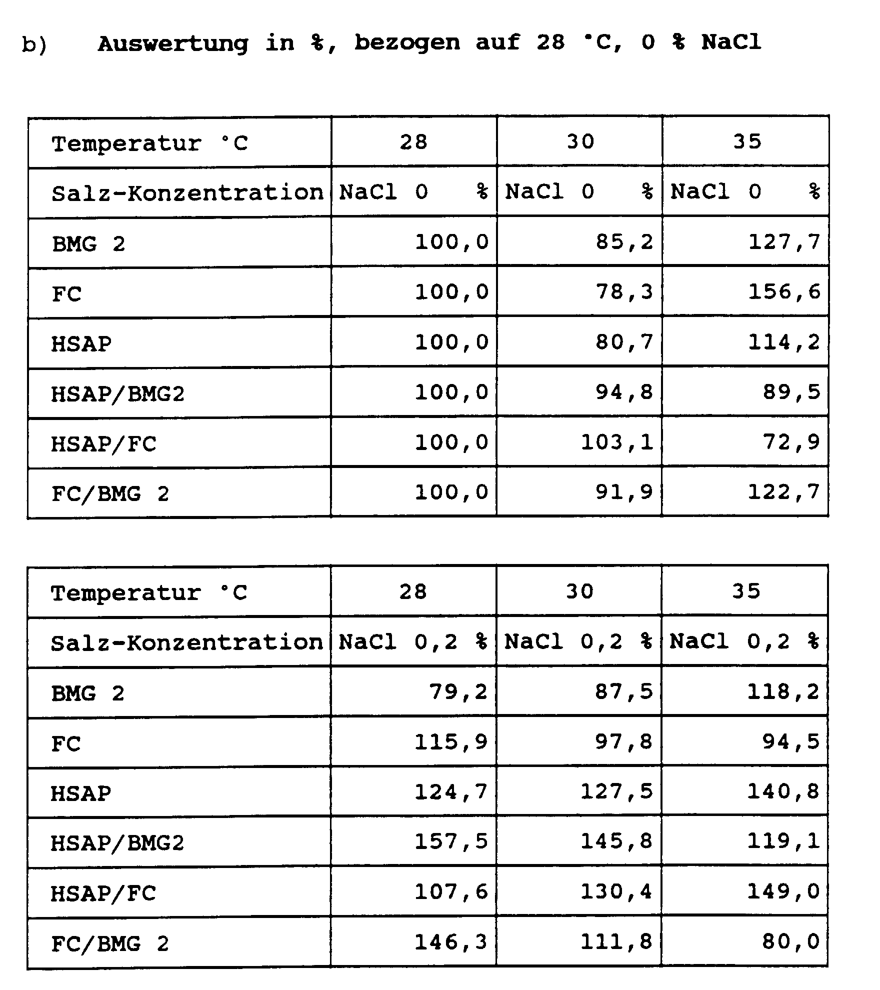

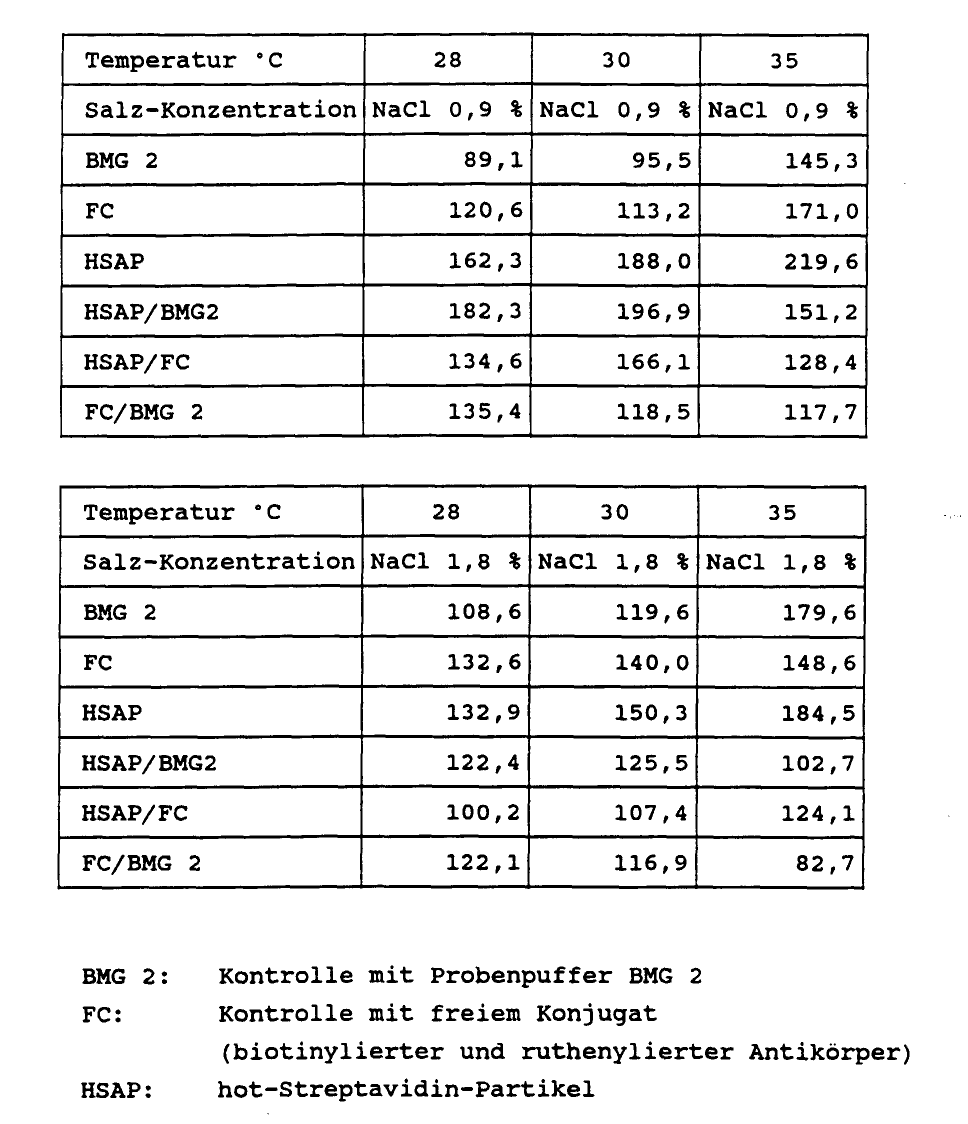

Eine Erniedrigung der Temperatur unter 20 °C erfordert einen Zusatz eines Alkalichlorids und einen pH-Wert von bevorzugt 7,25 - 7,75.

### Beispiel 3

### Nachweis von T₃

Am Beispiel eines Immunoassays zum Nachweis von Trijodthyronin (T₃) wird die erfindungsgemäße Testzusammensetzung BMG 1 mit der Testzusammensetzung des Standes der Technik (BMG O) verglichen:

Die Reagenzlösungen haben die folgende Zusammensetzung:

| Reagenz | BMG O | BMG 1 |
|---|---|---|
| KH₂PO₄ x2H₂O | 0,2 M | 0,2 M |
| H₃ PO₄ | - | - |
| Detergenz | Triton X-100 0,005 % | Polidocanol 0,1 % |
| | Tween 0,05 % | |
| Konservierungsmittel | NaN₃ 7,8 mM | Oxaban 0,1 % |
| Tripropylamin | 0,1 M | 0,1 M |
| KOH | 0,076 M | 0,076 M |
| pH | 7,5 | 7,5 |

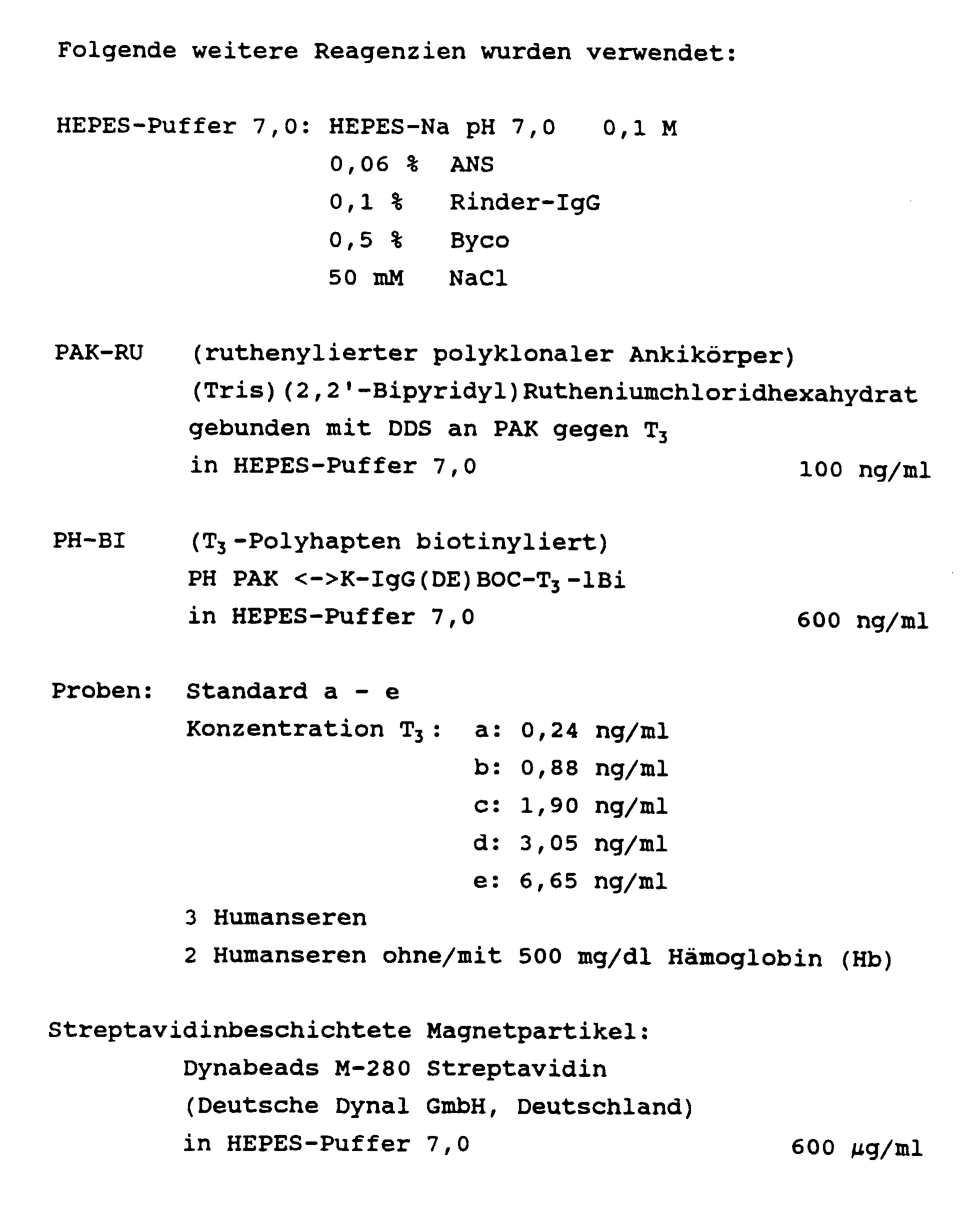

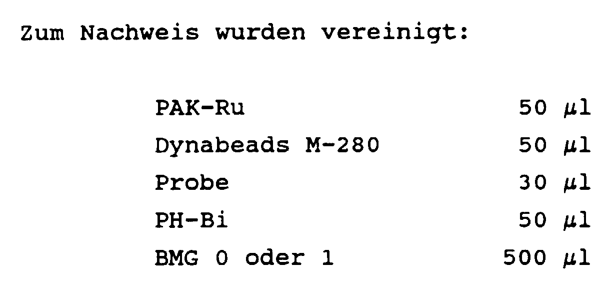

Dieses Gemisch wurde 16 Minuten bei 28 °C inkubiert, anschließend in die auf 28 °C temperierte Meßzelle überführt und die Partikel mit BMG 0 oder 1 je nach Testansatz gewaschen und darin vermessen. Es wurde eine Dreiecksrampenspannung angelegt. Die Meßspannung betrug 0,565 V und das PMT 720 mV.

Die Ergebnisse sind in der Tabelle 3 dargestellt. Es zeigt sich, daß die Hämoglobin-Störung, die im BMG 0 zu beobachten ist, im BMG 1 vermieden wird. Die untere Nachweisgrenze wird nicht beeinflußt.

**Tabelle 3:**

| | BMG O | BMG 1 |
|---|---|---|
| UNG (2s) | 0,3 ng/ml | 0,3 ng/ml |
| Humanserum ohne Hb | 100 % | 100 % |
| Humanserum mit Hb | 307 % | 106 % |
| UNG (2s) = Untere Nachweisgrenze | | |

### Beispiel 4

### Nachweis von HBsAg

Es wurde die erfindungsgemäße Testzusammensetzung BMG 1 im Vergleich zu BMG 0 am Beispiel eines Sandwich-Immunoassays zum Nachweis von HBsAg getestet. BMG 0 und 1 hatten die im Beispiel 3 angeführten Zusammensetzungen.

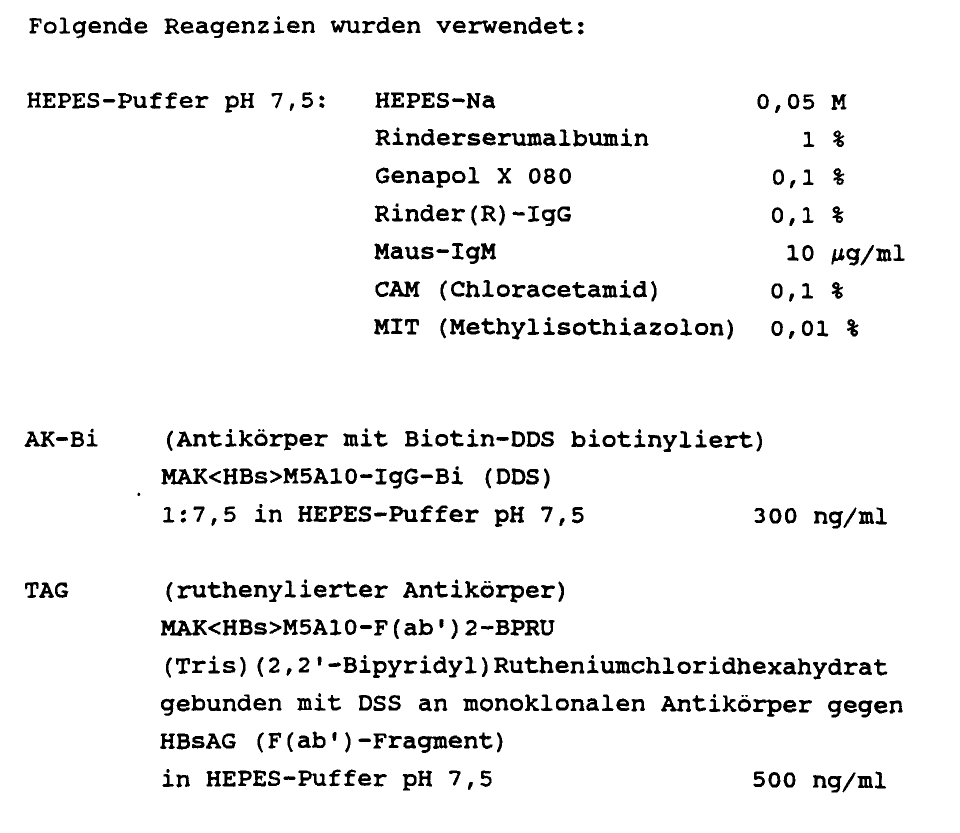

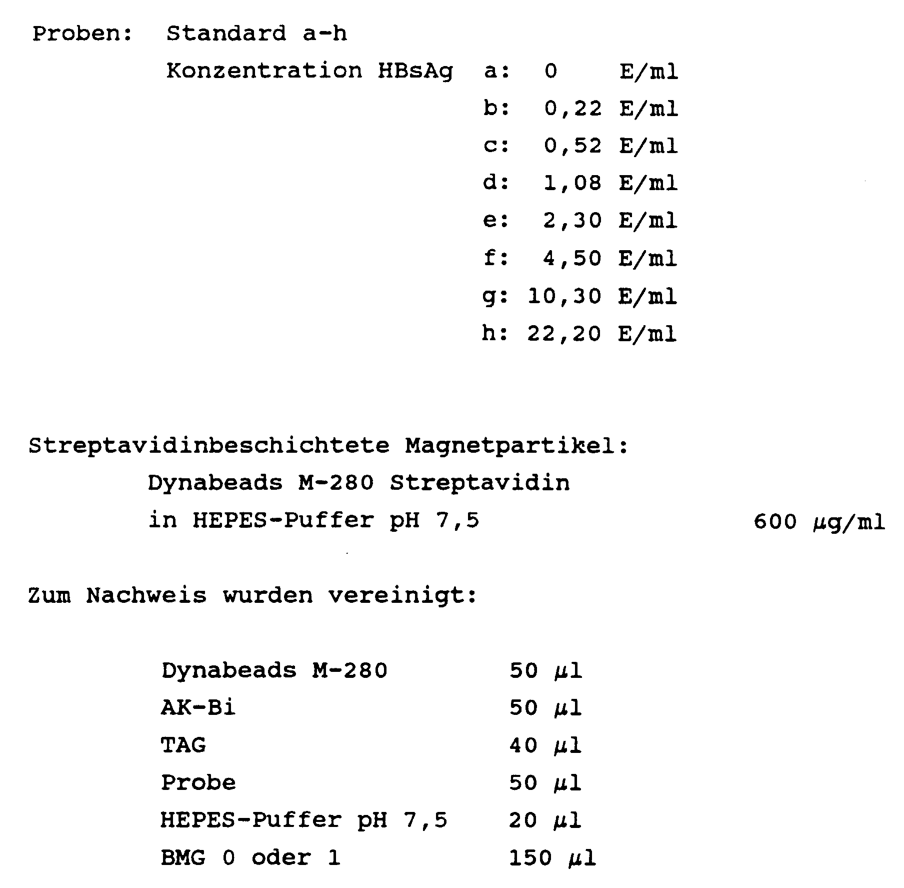

Dieses Gemisch wurde 16 Minuten bei 28 °C inkubiert, anschließend in die auf 28 °C temperierte Meßzelle überführt und die Partikel mit BMG 0 oder 1 gewaschen und darin vermessen.

Die Ergebnisse sind in der Tabelle 4 dargestellt. Durch die Verwendung von BMG 1 konnte die untere Nachweisgrenze deutlich verbessert werden. Ebenfalls wurde im Vergleich zu BMG 0 der VK erniedrigt. Das Verhältnis der Werte der Standardproben h zu a der Eichkurve ist bei BMG 1 erhöht, wodurch eine bessere Differenzierung der Eichkurve erreicht wird.

**Tabelle 4:**

| | BMG 0 | BMG 1 |
|---|---|---|
| UNG [E/ml] | 0,21 | 0,06 |
| Standard h/a | 40 | 78 |
| VK [%] | 6,9 | 4,3 |
| UNG = Untere Nachweisgrenze VK: = Variationskoeffizient | | |

### Beispiel 5

### Nachweis von TSH

Am Beispiel eines Sandwich-Immunoassays zum Nachweis von TSH (Thyroid-stimulierendes Hormon) werden die erfindungsgemäßen Testzusammensetzungen BMG 1 und BMG 1 ohne Oxaban im Vergleich zur Testzusammensetzung nach dem Stand der Technik BMG 0 verglichen. BMG 0 und 1 hatten die in Beispiel 3 beschriebene Zusammensetzung.

Thyroid-stimulierendes Hormon (TSH) wurde über einen Sandwich-Immunoassay bestimmt. Zur Ausführung kam hierbei ein Apparat, wie in Beispiel 1 beschrieben ist.

Zum Nachweis wurden vereinigt:

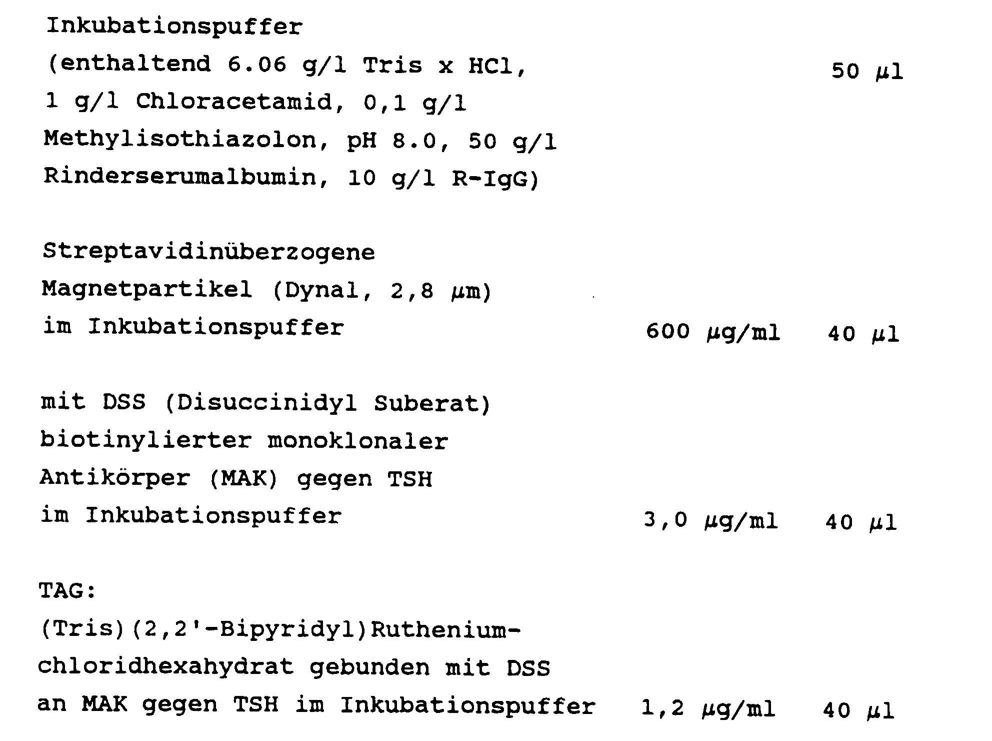

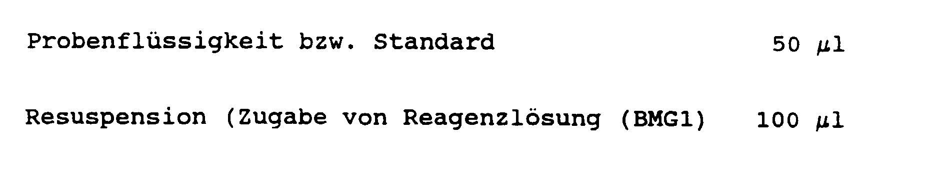

Dieses Gemisch wurde 16 Minuten bei Raumtemperatur (21°C) inkubiert und anschließend in die auf die gewünschte Meßtemperatur gebrachte Meßzelle überführt, die immobilisierten Partikel mit Reagenzlösung BMG1 gewaschen und in BMG1 vermessen.

Als Probe wurden die Standards a - e mit TSH-Konzentrationen von
- a:: 0 µU/ml
- b:: 0,39 µU/ml
- c:: 3,54 µU/ml
- d:: 12,4 µU/ml
- e:: 44,3 µU/ml
verwendet. Die Ergebnisse sind in der Tabelle 5 wiedergegeben. Durch BMG 1 wird im Vergleich zu BMG 0 eine bessere Nachweisgrenze erreicht. Durch Zusatz des Konservierungsmittels Oxaban wird die untere Nachweisgrenze weiter herabgesetzt. Eindeutig verbessert wird in beiden Beispielen (BMG 1 +/- Oxaban) ebenfalls der Variationskoeffizient.

**Tabelle 5:**

| | BMG 0 | BMG 1 | BMG 1 ohne Oxaban |
|---|---|---|---|
| UNG (2s) [mIU/ml] | 0,049 | 0,028 | 0,041 |
| VK [%] | 3,35 | 2,28 | 2,38 |

Die Abkürzungen haben die in Beispiel 4 genannten Bedeutungen.

## Patentansprüche

1. Verfahren zur Messung elektrochemilumineszenter Phänomene in einer Lösung oder aus einer an die Lösung angrenzenden festen Phase durch Inkontaktbringen einer zur Elektrochemilumineszenz fähigen Komponente, mit einem oxidierbaren Amin und Anlegen einer elektrischen Spannung zur Induktion der Elektrochemilumineszenz und Detektion der elektromagnetischen Strahlung, dadurch gekennzeichnet, daß die Lösung ein Detergenz ausgewählt aus der Gruppe Fettalkoholethoxylate, Plantaren und Octylglucosid oder ein Gemisch hiervon enthält.

2. Verfahren nach Anspruch 1, dadurch gekennzeichnet, daß als Fettalkoholethoxylat Polidocanol, C14-E09, Genapol oder C8-E09 verwendet wird.

3. Verfahren nach Anspruch 1 oder 2, dadurch gekennzeichnet, daß die Lösung ein oder mehrere Alkali- oder Erdalkalihalogenide enthält.

4. Verfahren nach Anspruch 1 bis 3, dadurch gekennzeichnet, daß der pH der Lösung zwischen 6,5 und 9,0 liegt.

5. Verfahren nach Anspruch 1 bis 4, dadurch gekennzeichnet, daß die Elektrochemilumineszenz durch Anlegen einer Rechtecks-Spannung von maximal 2,2 Volt oder einer Rampenspannung von maximal 3,0 Volt angeregt wird.

6. Verfahren zum Nachweis eines Analyten nach einem der Ansprüche 1 bis 5, dadurch gekennzeichnet, daß die zur Elektrochemilumineszenz fähige Komponente eine Markierung eines Analyten, eines Analytanalogen oder einer analytspezifischen Substanz ist und die detektierbare Strahlung ein Maß für die Anwesenheit des Analyten darstellt.

7. Reagenzlösung zur Messung elektrochemischer Phänomene enthaltend ein elektrochemisch oxidierbares Amin, das im oxidierten Zustand ein starkes Reduktionsmittel darstellt, dadurch gekennzeichnet, daß ein Detergenz ausgewählt aus der Gruppe Fettalkoholethoxylat, Plantaren und Octylglucosid oder ein Gemisch hiervon enthalten ist.

8. Reagenzlösung nach Anspruch 6, dadurch gekennzeichnet, daß das Detergenz in einer Konzentration von 0,001 bis 1% enthalten ist.

9. Reagenzlösung nach Anspruch 6 oder 7, dadurch gekennzeichnet, daß zusätzlich ein Alkalichlorid in einer Konzentration von 0,05 mmol/l bis 0,5 mol/l enthalten ist.

10. Reagenzlösung nach Anspruch 6 bis 8, dadurch gekennzeichnet, daß sie einen pH zwischen 6,5 und 9,0 aufweist.

## Claims

1. Method for measuring electrochemiluminescent phenomena in a solution or a solid phase contiguous with said solution by bringing a component capable of generating electrochemiluminscence into contact with an oxidizable amine or by applying an electrical voltage to induce electrochemilumiscence and detecting electromagnetic radiation. characterized in that the solution is a detergent selected from the group consisting of fat alcohol ethoxylate. plantaren or octylglucoside or a mixture thereof.

2. Method according to claim 1, characterized in that the fat alcohol ethoxylate is polidocanol, C14-E09, genapol or C8-E09.

3. Method according to claim 1 or 2, characterized in that the solution contains one or several alkali or earth alkali halogenides.

4. Method according to claim 1 to 3, characterized in that the pH of the solution ranges between 6.5 and 9.0.

5. Method according to claims 1 to 4, characterized in that electrochemiluminescence is achieved by applying a maximum square-wave voltage of 2.2 V or a maximum ramp voltage of 3.0 V.

6. Method for the detection of an analyte according to one of the claims 1 to 5, characterized in that the component capable of generating electrochemiluminescence is a label of an analyte, an analyte analog or an analyte-specific substance and the detectable radiation is a measure for the presence of the analyte.

7. Reagent solution for measuring electrochemical phenomena, containing an electrochemically oxidizable amine which is a strong reducing agent when oxidized, characterized in that it contains a detergent selected from the group consisting of fat alcohol ethoxylate. Plantaren and octylglucoside or a mixture thereof.

8. Reagent solution according to claim 6. characterized in that the detergent is contained in a concentration of 0.001 to 1 %.

9. Reagent solution according to claim 6 or 7. characterized in that in addition, an alkali chloride is contained in a concentration of 0.05 mmol/l up to 0.5 mol/l.

10. Reagent solution according to claims 6 to 8. characterized in that it has a pH between 6.5 and 9.0.

## Revendications

1. Procédé de mesure de phénomènes d'électrochimiluminescence dans une solution ou dans une phase solide jouxtant la solution, par mise en contact d'un composant, capable d'électrochimiluminescence, avec une amine oxydable et application d'une tension électrique pour l'induction de lélectrochimiluminescence et détection du rayonnement électromagnétique, caractérisé en ce que la solution contient un tensioactif choisi dans le groupe comprenant les éthylates d'alcools gras, les Plantarens et les octylglucosides ou un mélange de ces composés.

2. Procédé selon la revendication 1, caractérisé en ce que, en tant qu'éthylate d'alcool gras, on utilise du Polidocanol, du C14-E09, du Genapol ou du C8-E09.

3. Procédé selon la revendication 1 ou 2, caractérisé en ce que la solution contient un ou plusieurs halogénures alcalins ou alcalinoterreux.

4. Procédé selon les revendications 1 à 3, caractérisé en ce que le pH de la solution est compris entre 6.5 et 9.0.

5. Procédé selon les revendications 1 à 4, caractérisé en ce que l'électrochimiluminescence est provoquée par application d'une tension rectangulaire d'au plus 2,2 volts ou d'une tension en dents de scie d'au plus 3,0 volts.

6. Procédé de détection d'un analyte selon l'une quelconque des revendication 1 à 5, caractérisé en ce que le composant capable d'électrochimiluminescence est un marqueur d'analyte, un analogue d'analyte ou une substance spécifique à l'analyte et que le rayonnement détectable constitue une mesure de la présence de l'analyte.

7. Solution de réactif destinée à mesurer des phénomènes électrochimiques, contenant une amine oxydable par voie électrochimique, qui, à l'état oxydé, représente un puissant agent réducteur, caractérisée en ce qu'elle contient un tensioactif choisi dans le groupe comprenant les éthylates d'alcools gras, les Plantarens et les octylglucosides ou un mélange de ces composés.

8. Solution de réactif selon la revendication 6, caractérisée en ce que le tensioactif est présent en une concentration de 0,001 à 1 %.

9. Solution de réactif selon la revendication 6 ou 7, caractérisée en ce qu'elle contient en outre un chlorure alcalin en une concentration de 0,05 mmole/l à 0,5 mole/l.

10. Solution de réactif selon les revendications 6 à 8, caractérisée en ce qu'elle présente un pH compris entre 6,5 et 9,0.
